# EUROPÄISCHE PATENTSCHRIFT

(11) **EP 2 767 247 B1**
(45) Veröffentlichungstag und Bekanntmachung des Hinweises auf die Patenterteilung: **16.03.2016**
(21) Anmeldenummer: 14167876.3
(22) Anmeldetag: 22.06.2012
(51) Int. Cl.: A61B 17/80

(54) **Tibiaimplantat zur Straffung der Kniescheibenbänder**
Tibia implant for tightening of the patella ligaments
Implant tibial pour l'affinement du grasset

(30) Priorität: 26.07.2011 DE 102011079821
(43) Veröffentlichungstag der Anmeldung: 20.08.2014
(62) Teilanmeldung aus: 12173163.2
(73) Patentinhaber: Rita Leibinger GmbH & Co. KG, 78579 Neuhausen ob Eck (DE)
(72) Erfinder: Leibinger, Rita, 78570 Mühlheim (DE)
(74) Vertreter: DREISS Patentanwälte PartG mbB

(56) Entgegenhaltungen:
- WO-A1-2012/106323
- DE-A1- 3 224 265
- DE-A1-102005 037 141
- DE-A1-102006 047 663
- FR-A1- 2 887 760
- US-A- 6 086 593
- US-A1- 2010 076 564
- US-B2- 8 388 690
- US-B2- 8 430 930

## Beschreibung

Die Erfindung betrifft ein Tibiaimplantat zur Straffung der Kniescheibenbänder.

Aus der US 2010/0076564 A1 ist ein Implantat bekannt geworden, mit welchem insbesondere in der Veterinärmedizin die Bänder an einem Knie wieder gestrafft werden können. Dieses Dokument bildet die Basis für den Oberbegriff des Anspruchs 1.

Hierfür wird die Tibia im knienahen Bereich an der Vorderseite von oben bereichsweise aufgespaltet, so dass ein keilförmiger Einschnitt entsteht. Der Tibiaabschnitt darf aber nicht vollständig abgespalten werden. In den einschnitt wird das Implantat eingeschoben, so dass der Tibiaabschnitt nach vorne abgespreizt wird. Das Implantat ist ein Drahtgestellt, welches nach Art einer Leiterbahnstruktur aufgebaut ist und bezüglich des Keilwinkels flexibel ist. Nach dem Einschieben des Implantats in den Tibiaeinschnitt wird dieser so weit aufgebogen, dass sich die Seitenflächen des Implantats an den Schnittflächen des Einschnitts anlegen. Durch Umbiegen von Befestigungslaschen aus der Ebene der Schnittflächen in Richtung auf die Außenoberfläche der Tibia und des Tibiaabschnitts besteht nun die Möglichkeit, das Implantat an der Tibia mittels Schrauben zu befestigen.

Als nachteilig hat sich aber herausgestellt, dass das Implantat viel zu filigran ist und keine Kräfte aufnehmen kann und sich sehr schnell verformt. Außerdem verbindet oder verankert sich das Implantat nur mangelhaft mit dem Knochen.

Die DE 10 2005 037 141 A1 zeigt ein Implantat, welches einen offenporigen Metallschwamm als duroplastischen Knochenersatz aufweist. Die DE 32 24 265 A1 erwähnt ein Verfahren zur Herstellung eines Implantats, bei dem ein Modell aus durchgehend offenzelligem Material verwendet wird, wobei ein Modellmaterial mit einer räumlichen Gitter- oder Netzstruktur zur Anwendung kommt.

Weitere Dokumente betreffend Implantate sind US 5,766,251, US 8,430,930 B2, US 2011/0313532 A1, US 8,388,690 B2, FR 2 887 760 A1 und US 6,086,593 A1.

Der Erfindung liegt daher die Aufgabe zugrunde, ein Tibiaimplantat bereit zu stellen, welches formstabiler ist und welches besser mit dem Knochen verwächst.

Diese Aufgabe wird mit einem Tibiaimplantat gelöst, das die Merkmale des Anspruchs 1 aufweist.

Das erfindungsgemäße Implantat weist den wesentlichen Vorteil auf, dass es aufgrund des Schwammgebildes, welches eine beliebige Porengröße aufweisen kann, und insbesondere offenporig ist, sehr steif und formstabil ist und daher sehr hohe Kräfte aufnehmen und abstützen kann. Das Schwammgebilde besitzt bevorzugt eine netzartige, insbesondere regelmäßige Struktur.

Allgemein betrifft die vorliegende Erfindung insoweit ein Tibiaimplantat zur Straffung der Kniescheibenbänder, mit einem in Gebrauchslage nach unten sich verjüngenden vertikalen Querschnitt, einer in Längsrichtung der Tibia angeordneten Basisplatte, welche ein Schwammgebilde trägt, wobei die Tibia eine vertikale Schnittfläche eines vertikalen, knienahen Einschnitts mit zwei Schnittflächen aufweist und das Schwammgebilde auf den den Schnittflächen zugewandten Seiten an der Tibia anliegt und das Schwammgebilde zumindest abschnittsweise eine insbesondere regelmäßige Gitterstruktur aufweist.

Dadurch, dass eine Längserstreckung der Basisplatte längs einer Längsachse des Tibiaimplantats verläuft, ist die Basisplatte in Gebrauchslage - bei stehendem Mensch oder Tier - seitlich von der Schwammstruktur angeordnet, so dass sich sowohl das Schwammgebilde als auch die Basisplatte in Gebrauchslage - bei stehendem Menschen oder Tier - beide im Wesentlichen vertikal erstrecken.

Bei dem Einschnitt in der Tibia handelt es sich um einen "teilweisen" Einschnitt insoweit, als dieser am beim stehenden Menschen oder Tier oberen Ende der Tibia beginnt und sich in etwa in Längsrichtung der Tibia so weit erstreckt, dass ein Knochenstück vom Hauptteil der Tibia weg bewegt bzw. abgespreizt werden kann, so dass sich ein keilförmiger Spalt ergibt. In diesen kann dann das keilförmige Tibiaimplantat eingeschoben werden, so dass es mit zwei voneinander abgewandten Seiten an den einander gegenüber liegenden Seitenflächen des Einschnitts anliegt, wohingegen die Basisplatte und die Befestigungslaschen seitlich von dem Einschnitt angeordnet sind und dort den Knochen kontaktieren, und zwar auf der einen Seite den Hauptteil und auf der anderen Seite den abgespreizten teilabgetrennten Tibiaabschnitt.

Das Tibiaimplantat gestattet somit eine erhebliche Einsatzflexibilität, denn mit einem Satz mit einer relativ geringen Anzahl von Implantaten unterschiedlicher Größe und mit unterschiedlichen Keilwinkeln kann man alle möglichen Größenverhältnisse bei Mensch und Tier, insbesondere bei Hunden, befriedigen, und kann auch auf unterschiedliche Techniken beim Einbringen des Einschnittes reagieren.

Um das Tibiaimplantat schnell und einfach am Knochen befestigen zu können, ist die Basisplatte bzw. wenigstens eine vertikale Seitenfläche des Stützrahmens mit wenigstens zwei abragenden Befestigungslaschen versehen. Diese Befestigungslaschen sind flächig ausgebildet, so dass sie in der Laschenebene hohe Kräfte abstützen können. Dabei erstrecken sich die Befestigungslaschen in Gebrauchslage zur Tibia und zum teilabgetrennten Tibiaabschnitt. Eine optimale Anpassung wird dadurch erreicht, dass die Befestigungslaschen horizontal und/oder zur Horizontalen geneigt vom Stützrahmen abragen. Außerdem können die Befestigungslaschen eine oder mehrere Aufnahmeöffnungen für Befestigungsmittel, wie Schrauben oder dergleichen, aufweisen. Eine Verhakung ist ebenfalls möglich. Eine schnelle individuelle Anpassung des Implantats an die jeweilige Knochenform wird dadurch erreicht, dass die Befestigungslaschen biegbar und an die Tibia anpassbar vom Stützrahmen abragen.

Dabei ist die Basisplatte vorteilhaft ein Teil eines umlaufenden, das Schwammgebilde umschließenden Stützrahmens. Einerseits trägt der umlaufende Stützrahmen das Schwammgebilde, andererseits dient der Stützrahmen als stabiler Abstandshalter für den Tibiaabschnitt und hält diesen in der gewünschten abgespreizten Lage, so dass die Bänder ihre neu geschaffene Straffung beibehalten.

Bei einem bevorzugten Ausführungsbeispiel weist das Schwammgebilde eine räumliche Struktur auf. Hierdurch wird gewährleistet, dass die Abstützkräfte in das Innere des Implantats eingeleitet werden, ohne dass das Schwammgebilde beschädigt wird.

Dabei erstreckt sich die Gitterstruktur über mehrere parallele Ebenen. Selbst dann, wenn die Gitterstruktur in einer Ebene eine Fehlstelle aufweist, bleibt die Steifigkeit und Festigkeit erhalten, da die Abstützung durch die weiteren Ebenen unterstützt und gewährleistet wird.

Eine bevorzugte Ausgestaltung des erfindungsgemäßen Tibiaimplantats sieht vor, dass zwei parallele Gitterstrukturebenen um eine halbe Gitterbreite in der x- und/oder in der y-Achse verschoben sind. Dabei sind die Gitterstrecken zwischen den Gitterpunkten in Richtung auf die parallel dazu liegende Gitterstrukturebene abgeknickt, so dass sie sich in ihren Knickstellen berühren. Die abgeknickten Gitterstrecken spannen nicht nur die Gitterstrukturebene auf sondern versteifen dieses auch gegenüber der parallelen Ebene. Eine detaillierte Beschreibung findet sich weiter unten, wo auf die Zeichnung Bezug genommen wird.

Bevorzugt ist das erfindungsgemäße Tibiaimplantat aus Metall, z.B. Edelstahl oder Titan, oder aus Kunststoff, z.B. PEEK, hergestellt ist. Legierungen der Metallwerkstoffe oder Mischungen der Kunststoffe, auch Materialbeimischungen, wie Glasfaser oder Kohlefaser sind denkbar.

Um die Verankerung des Implantats im Knochen zu fördern, weist das Tibiaimplantat eine das Anwachsen von Knochenmasse unterstützende Beschichtung, z.B. Hydroxylapatit, auf. Außerdem wird das Schwammgebilde von Dornen überragt. Diese dringen in die Knochenoberfläche ein und verhindern ein Verrutschen des Tibiaimplantats am Knochen. Dabei sind die Dorne im Bereich der Keilschneide kleiner sind und/oder ragen weniger weit aus dem Schwammgebilde ab als am gegenüber liegenden breiteren Ende. Sehr hohe Kräfte können in bevorzugter Weise dadurch aufgenommen werden, dass die Dorne sich vom einen Schwammgebilde durch das Implantat und dessen Innenraum hindurch zum anderen Schwammgebilde erstrecken und dieses nach außen überragen, so dass die Kräfte vom Tibiaabschnitt direkt auf die Tibia übertragen werden.

Ein schnelles Einwachsen des Tibiaimplantats wird vorteilhaft dadurch gefördert, dass der Stützrahmen Durchbrüche aufweist. Durch diese Durchbrüche kann Knochenmasse in das Tibiaimplantat eingeführt werden, so dass der zwischen den Schwammgebilden sich befindende Innenraum schnell zuwächst.

Ein vorteilhaftes Herstellungsverfahren sieht vor, dass das Tibiaimplantat mittels eines rapid manufacturing Verfahrens hergestellt wird. Das Implantat kann dadurch ohne große Konstruktionsänderungen in mehreren Größen hergestellt werden.

Weitere Vorteile, Merkmale und Einzelheiten der Erfindung ergeben sich aus den Unteransprüchen sowie der nachfolgenden Beschreibung, in der unter Bezugnahme auf die Zeichnung ein besonders bevorzugtes Ausführungsbeispiel im Einzelnen beschrieben ist.

In der Zeichnung zeigen:
- Figur 1: eine Frontansicht auf die Vorderseite eine Knies mit in der Tibia sich befindendem Implantat;
- Figur 2: eine Seitenansicht des Knies in Richtung des Pfeils II gemäß Figur 1;
- Figur 3: eine erste perspektivische Ansicht des Tibiaimplantats;
- Figur 4: eine schematische Darstellung des Schwammgebildes;
- Figur 5: eine zweite perspektivische Ansicht des Tibiaimplantats;
- Figur 6: eine Ansicht in Richtung des Pfeils VI gemäß Figur 1 auf die Schneide des Tibiaimplantats;
- Figur 7a: ein Gitterelement in perspektivischer Ansicht; und
- Figur 7b: ein aus mehreren Gitterelementen aufgebautes Netz.

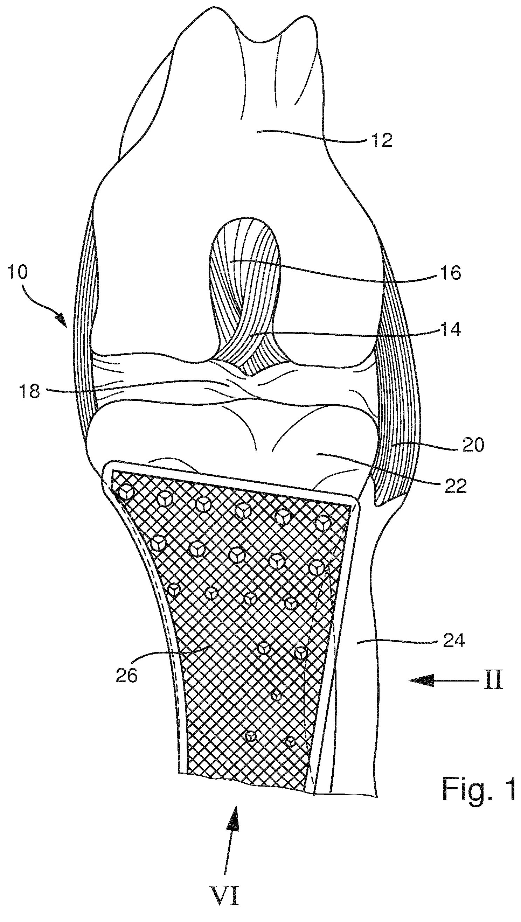

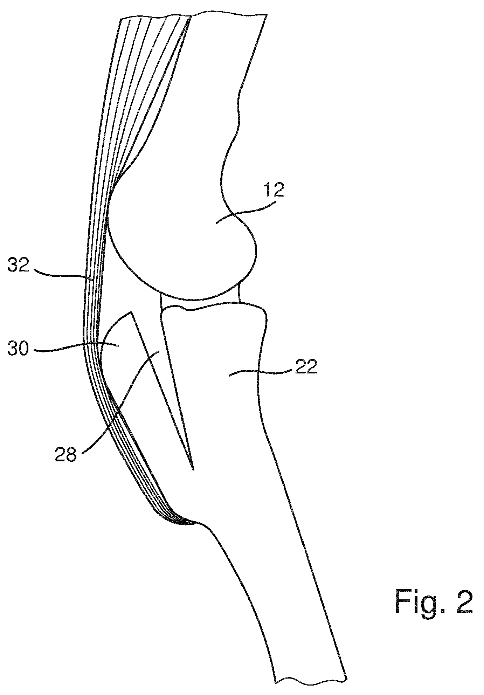

Die Figur 1 zeigt eine Frontansicht auf die Vorderseite eine Knies 10, wobei das untere Ende des Femurs 12. Es sind andeutungsweise das vordere Kreuzband 14 und das hintere Kreuzband 16 sowie die Menisken 18 und das Seitenband 20 dargestellt. Neben der Tibia 22 ist die Fibula 24 zu erkennen. Im knienahen Bereich der Tibia 22 ist ein Tibiaimplantat 26 erkennbar, das einen im Wesentlichen trapezförmigen Querschnitt mit einer konkav geformten Seite aufweist. Dieses Tibiaimplantat 26 befindet sich in einem Einschnitt 28 der Tibia 22, der, wie aus Figur 2 erkennbar, im Wesentlichen vertikal von oben in die Tibia 22 derart eingebracht wird, dass ein Tibiaabschnitt 30 entsteht, dessen unteres Ende noch mit der Tibia 22 verbunden ist. In diesen V-förmigen Einschnitt 28 wird das Tibiaimplantat 26 von der Seite eingesetzt. Durch das Abspreizen des Tibiaabschnitts 30 wird das vordere Band 32 gestrafft.

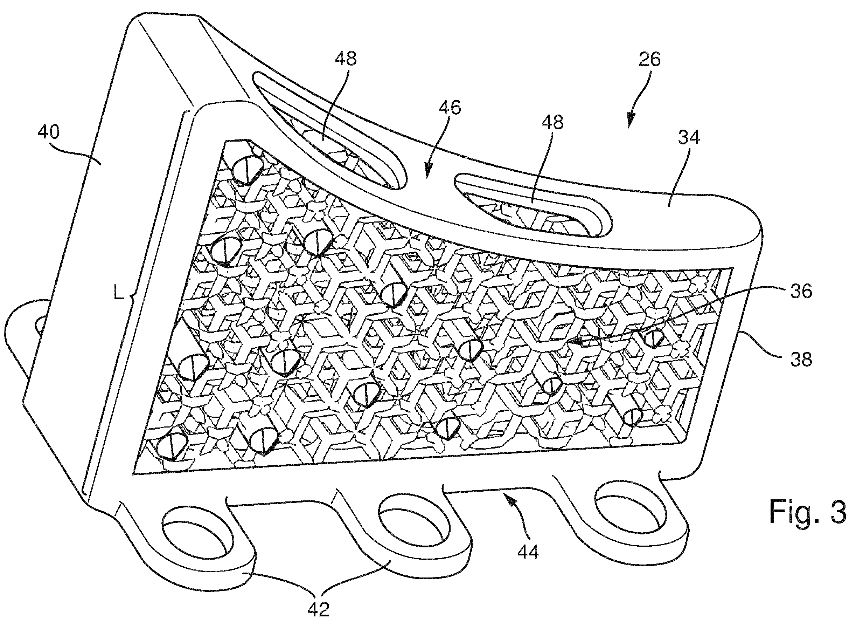

Die Figur 3 zeigt eine perspektivische Ansicht des Tibiaimplantats 26, das einen umlaufenden Stützrahmen 34 und ein dazwischen sich befindendes Schwammgebilde 36 aufweist. Das Tibiaimplantat 26 ist keilförmig ausgebildet und weist eine Keilschneide 38 auf. Die gegenüber liegende Seite 40 besitzt nicht nur eine größere Breite B sondern auch eine größere Länge L. Das Tibiaimplantat 26 weist demnach eine Trapezform auf, wobei die mehrere Befestigungslaschen 42 aufweisende Basisplatte 44 eben und die gegenüber liegende Seite 46 konkav ausgebildet sind. Außerdem ist erkennbar, dass diese konkave Seite 46 mit zwei Durchbrüchen 48 versehen ist, durch welche Knochenmaterial ins Innere des Tibiaimplantats 26 einfüllbar ist. Die Basisplatte 44 kann bei einem alternativen Ausführungsbeispiel auch konkav ausgebildet sein.

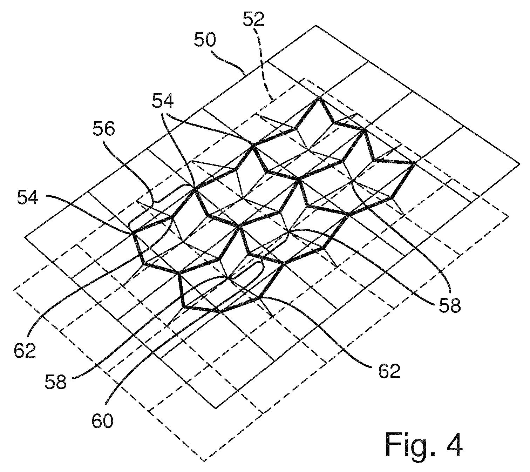

Das Schwammgebilde 36 erstreckt sich über wenigstens zwei Ebenen 50 und 52, was in Figur 4 deutlich zum Ausdruck kommt. Die Ebene 50 ist in der Figur 4 oben und die Ebene 52 darunter angeordnet. Außerdem ist das Gitter in der Ebene 50 um eine halbe Gitterbreite in der x-Richtung und in der y-Richtung verschoben. Die zwischen den Gitterpunkten 54 verlaufenden Gitterstrecken 56 sind in Richtung auf das parallel dazu liegende Schwammgebilde, d.h. die Ebene 52 abgeknickt. Entsprechend sind die zwischen den Gitterpunkten 58 verlaufenden Gitterstrecken 60 in Richtung auf das parallel dazu liegende Schwammgebilde, d.h. die Ebene 50 abgeknickt. Dabei berühren sich die Gitterstrecken 56 und 60 in den Knickstellen 62. Das Schwammgebilde 36 wird somit von einem räumlichen Geflecht gebildet, da eine Vielzahl von Öffnungen aufweist, in welche der Knochen einwachsen kann.

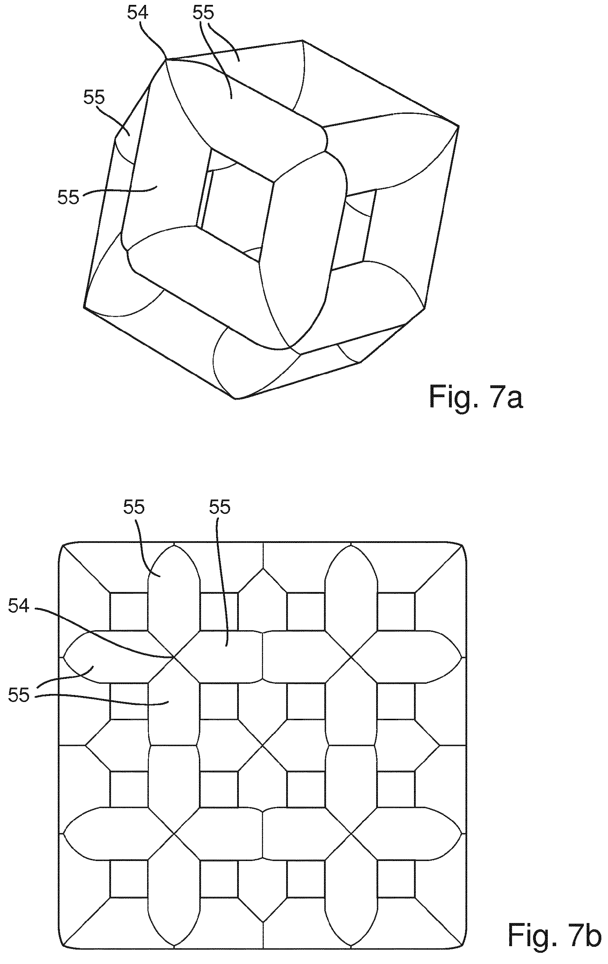

Die Figur 7a zeigt ein einzelnes Gitterelement, welches zwischen 3 Ebenen aufgebaut ist. Vom Gitterpunkt 54 ragen in gleichmäßigen Winkelabständen vier Gitterstäbe 55 zur nächsten, parallelen Ebene ab. Das Gitterelement wird aus zwölf gleichen Gitterstäben 55 aufgespannt. In allen Gitterstäben 55 ist die neutrale Faser gleich. Die Figur 7b zeigt ein aus mehreren Gitterelementen aufgebautes Netz, das sich in der x-y-Ebene im 90°-Winkel erstreckt. In der z-Ebene ist es um 45° verschoben.

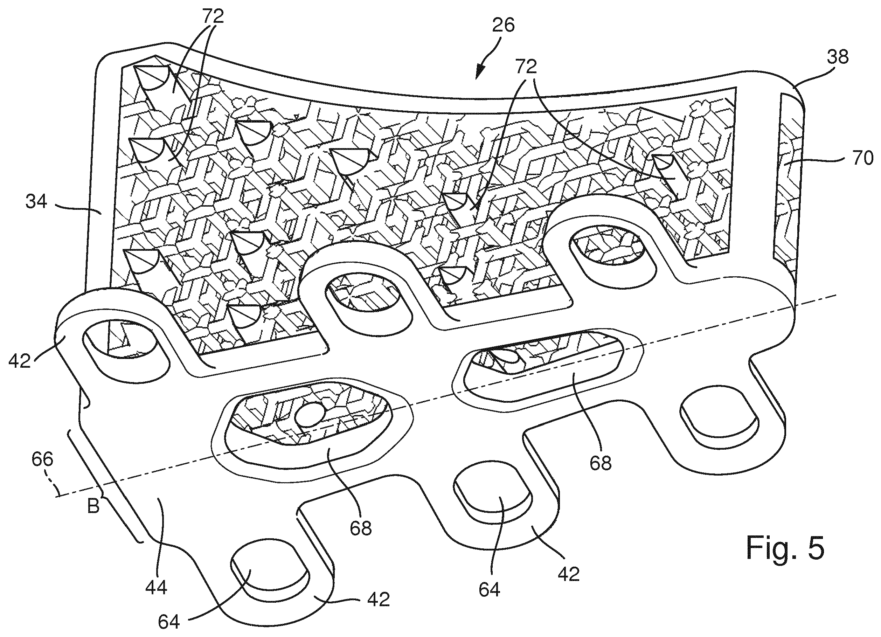

Die Figur 5 zeigt ebenfalls eine perspektivische Ansicht auf das Tibiaimplantat 26, wobei deutlich die Befestigungslaschen 42 erkennbar sind. Sie ragen in der Ebene der Basisplatte 44 vom Stützrahmen 34 ab und weisen jeweils eine Aufnahmeöffnung 64 für eine nicht dargestellte Befestigungsschraube (Knochenschraube) auf, die als quer zur Längsachse 66 des Tibiaimplantats 26 liegendes Langloch ausgebildet ist. Die Basisplatte 44 ist ebenfalls mit Durchbrüchen 68 versehen, so dass auch durch die Basisplatte 44 Knochenmaterial ins Innere des Tibiaimplantats 26 einfüllbar ist.

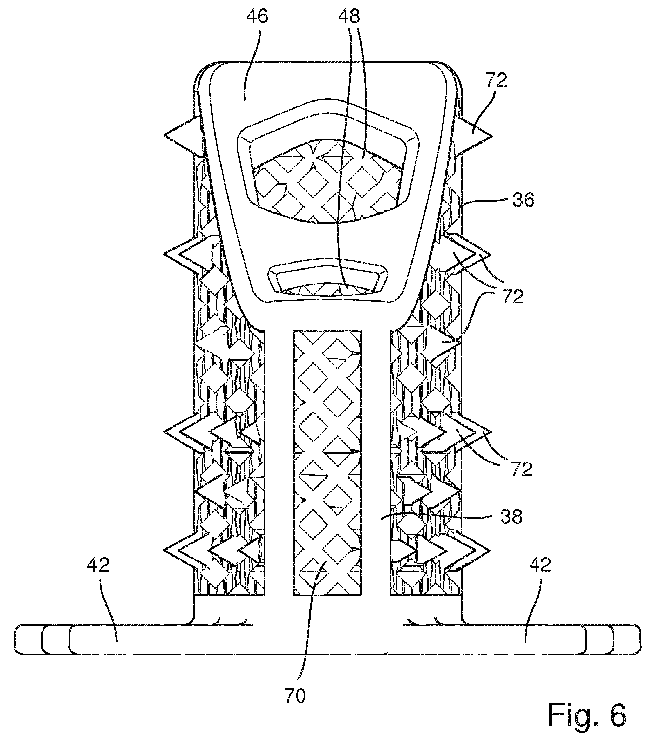

Die Figur 6 zeigt das Tibiaimplantat 26 in Richtung des Pfeils VI gemäß Figur 1, so dass deutlich der Durchbruch 70 in der Keilschneide 38 zu sehen ist. Dieser Durchbruch 70 dient ebenfalls zum Einfüllen von Knochenmaterial ins Innere des Tibiaimplantats 26. Es sind jedoch auch aus dem Schwammgebilde 36 herausragende Dorne 72 erkennbar, die die Außenkontur des Tibiaimplantats 26 überragen. Diese Dorne 72 durchsetzen das gesamte Tibiaimplantat 26, so dass die vom Tibiaabschnitt 30 eingeleiteten Kräfte direkt auf die Tibia 22 übertragen werden. Das Tibiaimplantat 26 und insbesondere das Schwammgebilde 36 wird nicht oder nur minimal belastet. Die Dorne 72 sind im Bereich der Keilschneide 38 nicht nur kürzer sondern weisen auch einen kleineren Durchmesser auf, wodurch das Einschieben des Tibiaimplantats 26 in den Einschnitt 28 erleichtert wird.

## Patentansprüche

1. Tibiaimplantat (26) zur Straffung der Kniescheibenbänder (32), mit einem in Gebrauchslage nach unten sich verjüngenden vertikalen Querschnitt, **dadurch gekennzeichnet, dass** es ein Schwammgebilde (36), welches zumindest abschnittsweise eine Gitterstruktur aufweist, mit zwei Seiten aufweist, die an zwei Schnittflächen eines knienahen, im Wesentlichen vertikal von oben in die Tibia (22) eingebrachten Einschnitts (28) anlegbar sind, dass es eine in Längsrichtung der Tibia (22) anordenbare Basisplatte (44) aufweist, welche das Schwammgebilde (36) trägt, wobei sich eine Längserstreckung der Basisplatte (44) längs einer Längsachse (66) des Tibiaimplantats (26) erstreckt und wobei die Basisplatte (44) vorzugsweise mit wenigstens zwei abragenden Befestigungslaschen (42) versehen ist.

2. Tibiaimplantat nach Anspruch 1, **dadurch gekennzeichnet, dass** die Basisplatte (44) Teil eines umlaufenden, das Schwammgebilde (36) umgebenden Stützrahmens (34) ist.

3. Tibiaimplantat nach einem der vorhergehenden Ansprüche, **dadurch gekennzeichnet, dass** das Schwammgebilde (36) offenporig ist und/oder eine räumliche Struktur aufweist und/oder von Dornen (72) überragt wird.

4. Tibiaimplantat nach einem der vorhergehenden Ansprüche, **dadurch gekennzeichnet, dass** die Gitterstruktur sich in einer Ebene erstreckt und das Schwammgebilde (36) mehrere parallele Ebenen (50, 52) derartiger Gitterstrukturen aufweist.

5. Tibiaimplantat nach einem der vorhergehenden Ansprüche, **dadurch gekennzeichnet, dass** zwei parallele Gitterstrukturebenen um eine halbe Gitterbreite in der x- und/oder in der y-Achse verschoben sind.

6. Tibiaimplantat nach Anspruch 5, **dadurch gekennzeichnet, dass** die Gitterstruktur von Gitterstrecken (56, 60) und Gitterpunkten (54, 58) gebildet wird, wobei die Gitterstrecken (56, 60) zwischen den Gitterpunkten (54, 58) in Richtung auf die parallel dazu liegende Gitterstrukturebene abgeknickt sind, so dass die Ebenen sich in ihren Knickstellen (62) berühren.

7. Tibiaimplantat nach einem der vorhergehenden Ansprüche, **dadurch gekennzeichnet, dass** die Befestigungslaschen (42) horizontal und/oder zur Horizontalen geneigt vom Stützrahmen (34) abragen.

8. Tibiaimplantat nach einem der vorhergehenden Ansprüche, **dadurch gekennzeichnet, dass** die Befestigungslaschen (42) eine oder mehrere Aufnahmeöffnungen (64) für Befestigungsmittel, wie Schrauben oder dergleichen, aufweisen.

9. Tibiaimplantat nach einem der vorhergehenden Ansprüche, **dadurch gekennzeichnet, dass** es aus Metall, z.B. Edelstahl oder Titan, oder aus Kunststoff, z.B. PEEK, hergestellt ist und/oder eine das Anwachsen von Knochenmasse unterstützende Beschichtung, z.B. Hydroxylapatit, aufweist.

10. Tibiaimplantat nach Anspruch 3, **dadurch gekennzeichnet, dass** die Dorne (72) sich in Richtung des verjüngenden Querschnitts verkleinern und/oder weniger weit aus dem Schwammgebilde (36) abragen.

11. Tibiaimplantat nach Anspruch 3 oder 10, **dadurch gekennzeichnet, dass** die Dorne (72) sich von der einen, an der Tibia (22) anliegenden Seite durch das Tibiaimplantat (26) hindurch zur anderen, an der Tibia (22) anliegenden Seite erstrecken.

12. Tibiaimplantat nach einem der vorhergehenden Ansprüche, **dadurch gekennzeichnet, dass** die Basisplatte (44) oder ein das Schwammgebilde (36) umgebender Stützrahmen (34) Durchbrüche (48, 68, 70) aufweist.

13. Verfahren zur Herstellung eines Tibiaimplantats nach einem der vorhergehenden Ansprüche, **dadurch gekennzeichnet, dass** es mittels eines rapid manufacturing Verfahrens hergestellt wird, indem mehrere zumindest abschnittsweise insbesondere regelmäßige Gitterstrukturebenen (50, 52) mit Gitterstrecken (56, 60) und Gitterpunkten (54, 58) hergestellt werden, wobei die Gitterstrecken (56, 60) einer Gitterstrukturebene (50, 52) in Richtung auf die parallel dazu liegende Gitterstrukturebene (50, 52) abgeknickt sind, und die Gitterstrecken (56) einer Gitterstrukturebene (50) die zu diesen benachbarten Gitterstrecken (60) einer benachbarten Gitterstrukturebene (52) in ihren Knickstellen (62) berühren.

## Claims

1. A tibia implant (26) for tightening the patella ligaments (32), having a vertical cross section tapering downward in the position for use, **characterized in that** it has a spongelike construction (36), which at least in some portions has a grid structure, having two sides, which can be placed against two cut faces of an incision (28) made near the knee, essentially vertically from above, into the tibia (22); that it has a base plate (44) that can be located in the longitudinal direction of the tibia (22) and that carries the spongelike construction (36), and a longitudinal extent of the base plate (44) extends along a longitudinal axis (66) of the tibia implant (26), and the base plate (44) is preferably provided with at least two protruding fastening tabs (42).

2. The tibia implant of claim 1, **characterized in that** the base plate (44) is part of an encompassing support frame (34) that surrounds the spongelike construction (36).

3. The tibia implant of one of the foregoing claims, **characterized in that** the spongelike construction (36) is open-pored and/or has a three-dimensional structure and/or has spikes (72) protruding beyond it.

4. The tibia implant of one of the foregoing claims, **characterized in that** the grid structure extends in a plane, and the spongelike construction (36) has a plurality of parallel planes (50, 52) of such grid structures.

5. The tibia implant of one of the foregoing claims, **characterized in that** two parallel grid structure planes are offset by one-half a grid width in the X and/or Y axis.

6. The tibia implant of claim 5, **characterized in that** the grid structure is formed of grid lengths (56, 60) and grid points (54, 58), and the grid lengths (56, 60) between the grid points (54, 58) are bent in the direction of the grid structure plane located parallel to them, so that the planes touch one another at their bending points (62).

7. The tibia implant of one of the foregoing claims, **characterized in that** the fastening tabs (42) protrude, inclined horizontally and/or to the horizontal, from the support frame (34).

8. The tibia implant of one of the foregoing claims, **characterized in that** the fastening tabs (42) have one or more receptacle openings (64) for fastening means, such as screws or the like.

9. The tibia implant of one of the foregoing claims, **characterized in that** it is produced from metal, such as special steel or titanium, or from plastic, such as PEEK, and/or has a coating, such as hydroxylapatite, that promotes the growth of bone mass thereon.

10. The tibia implant of claim 3, **characterized in that** the spikes (72), in the direction of the tapering cross section, decrease in size and/or protrude less far from the spongelike construction (36).

11. The tibia implant of claim 3 or 10, **characterized in that** the spikes (72) extend through the tibia implant (26) from the one side that contacts the tibia (22) to the other side that contacts the tibia (22).

12. The tibia implant of one of the foregoing claims, **characterized in that** the base plate (44) or a support frame (34) surrounding the spongelike construction (36) has apertures (48, 68, 70).

13. A method for producing a tibia implant of one of the foregoing claims, **characterized in that** the implant is produced by means of a rapid manufacturing process, **in that** a plurality of grid structure planes (50, 52) which at least in some portions are in particular regular and which have grid lengths (56, 60) and grid points (54, 58) are produced, and the grid lengths (56, 60) of one grid structure plane (50, 52) are bent in the direction of the grid structure plane (50, 52) located parallel to it, and the grid lengths (56) of one grid structure plane (50) touch the grid lengths (60) adjacent to them of an adjacent grid structure plane (52) at its bending points (62).

## Revendications

1. Implant tibial (26) destiné à raffermir les ligaments rotuliens (32), ayant une section verticale se rétrécissant vers le bas en position d'utilisation, **caractérisé par le fait qu'**il présente une formation spongieuse (36) présentant, au moins par sections, une structure en grille, avec deux faces qui peuvent être appliquées sur deux surfaces de coupe d'une entaille (28) proche du genou et réalisée pour l'essentiel verticalement d'en haut dans le tibia (22), qu'il présente une plaque de base (44) qui est apte à être disposée dans la direction longitudinale du tibia (22) et qui supporte ladite formation spongieuse (36), une extension longitudinale de la plaque de base (44) s'étendant suivant un axe longitudinal (66) de l'implant tibial (26) et la plaque de base (44) étant pourvue de préférence d'au moins deux languettes de fixation (42) faisant saillie.

2. Implant tibial selon la revendication 1, **caractérisé par le fait que** ladite plaque de base (44) fait partie d'un cadre d'appui (34) périphérique entourant la formation spongieuse (36).

3. Implant tibial selon l'une quelconque des revendications précédentes, **caractérisé par le fait que** la formation spongieuse (36) est à pores ouverts et/ou présente une structure spatiale et/ou est surmontée d'épines (72).

4. Implant tibial selon l'une quelconque des revendications précédentes, **caractérisé par le fait que** la structure en grille s'étend dans un plan et que la formation spongieuse (36) présente une pluralité de plans parallèles (50, 52) de telles structures en grille.

5. Implant tibial selon l'une quelconque des revendications précédentes, **caractérisé par le fait que** deux plans de structure en grille sont déplacés d'une demi-largeur de grille dans l'axe x et/ou dans l'axe y.

6. Implant tibial selon la revendication 5, **caractérisé par le fait que** la structure en grille est constituée par des segments de grille (56, 60) et des points de grille (54, 58), les segments de grille (56, 60) entre les points de grille (54, 58) étant pliés vers le plan de structure en grille situé parallèlement à ceci, de sorte que les plans se touchent sur leurs points de pliage (62).

7. Implant tibial selon l'une quelconque des revendications précédentes, **caractérisé par le fait que** les languettes de fixation (42) font saillie dudit cadre d'appui (34) horizontalement et/ou de manière à être inclinées vers l'horizontale.

8. Implant tibial selon l'une quelconque des revendications précédentes, **caractérisé par le fait que** les languettes de fixation (42) présentent une ou plusieurs ouvertures de réception (64) pour des moyens de fixation, tels que vis ou similaires.

9. Implant tibial selon l'une quelconque des revendications précédentes, **caractérisé par le fait qu'**il est réalisé en métal, par exemple en acier spécial ou en titane, ou en matière plastique, par exemple en PEEK, et/ou présente un revêtement, par exemple de l'hydroxyapatite, soutenant **le fait que** de la masse osseuse s'attache.

10. Implant tibial selon la revendication 3, **caractérisé par le fait que** les épines (72) diminuent en direction de la section se rétrécissant et/ou sont moins en saillie de la formation spongieuse (36).

11. Implant tibial selon la revendication 3 ou 10, **caractérisé par le fait que** les épines (72) s'étendent depuis l'une des faces qui s'applique sur le tibia (22), à travers l'implant tibial (26), vers l'autre face s'appliquant sur le tibia (22).

12. Implant tibial selon l'une quelconque des revendications précédentes, **caractérisé par le fait que** la plaque de base (44) ou un cadre d'appui (34) entourant la formation spongieuse (36) présente des percées (48, 68, 70).

13. Procédé de fabrication d'un implant tibial selon l'une quelconque des revendications précédentes, **caractérisé par le fait qu'**il est fabriqué au moyen d'un procédé de fabrication rapide en réalisant une pluralité de plans de structure en grille (50, 52) au moins par sections en particulier réguliers, ayant des segments de grille (56, 60) et des points de grille (54, 58), les segments de grille (56, 60) d'un plan de structure en grille (50, 52) étant pliés vers le plan de structure en grille (50, 52) situé parallèlement à ceci, et les segments de grille (56) d'un plan de structure en grille (50) touchant les segments de grille (60) d'un plan de structure en grille (52) voisin, qui sont voisins de ceux-ci, sur leurs points de pliage (62).
